Europäisches Patentamt

(19) European Patent Office

Office européen des brevets

(11) EP 0 917 826 A1

(12) **EUROPEAN PATENT APPLICATION**
published in accordance with Art. 158(3) EPC

(43) Date of publication:
26.05.1999 Bulletin 1999/21

(51) Int. Cl.$^6$: **A23L 1/305**

(21) Application number: 98921495.2

(22) Date of filing: 26.05.1998

(86) International application number:
PCT/ES98/00149

(87) International publication number:
WO 98/53709 (03.12.1998 Gazette 1998/48)

(84) Designated Contracting States:
DE ES FR GB IT NL PT

(30) Priority: 28.05.1997 ES 9701157

(71) Applicant: Schwartz Riera, Simon
08330 Premia de Mar (ES)

(72) Inventors:
• SCHWARTZ RIERA, Simón
E-08330 Premia de Mar (ES)

• ARBOS VIA, Maria Antonia
E-08970 Sant Joan Despi (ES)
• GARCIA ARUMI, Elena
E-08024 Barcelona (ES)

(74) Representative:
Urizar Anasagasti, José Antonio et al
Puerto Rico 6A, Bajo
28016 Madrid (ES)

(54) **AMINOACID FORMULATIONS FOR THIRD AGE PERSONS AND PROCESS FOR CALCULATING SUCH FORMULATIONS**

(57) Aminoacid formulations used in artificial diets by enteral or parenteral administration for third age persons, the composition of which for each 100 g of total aminoacids is: 11-17 g of L-glutamic acid; 6-8 g of L-serine; 4-10g of glycin; 2-3,50 g of L-histidine; 3,50-5 g of L-treonine; 5-6.50 g of L-alanine; 4.50-7 of L-arginine; 8.25-10 g of L-proline; 1.50-4.50 g of L-tyrosine; 5.50-7 g of L-valine; 2-3.50 g of L-methionine; 0.25-2.25 g of L-cysteine; 5.75-7.25 g of L-isoleucine; 9-10.75 g of L-leucine; 4-6 g of L-phenylalanine; 1-2.50 g of L-trytophane; 7-8.50 g of L-lysine; additionally L-anserine and L-aspartic acid in diets by enteral nutritional administration; as well as a process for calculating said aminoacid formulations.

EP 0 917 826 A1

**Description**

Object of the Invention

The present invention relates to amino acids formulations for artificial diets for elder persons. Also, the present invention relates to a procedure for the calculation of required concentration for each amino acid on parental or enteral administration as well as therapeutic use of amino acids formulations in artificial diets for elder persons.

Background of the Invention

Recently the number of elder patients more than 65 yearss old in hospitals has increased considerably. Many of them receive conventional artificial nutrition without consideration to their specific needs. Until now, no aminoacids formulations for enteral or parenteral nutritional administration, directed to elder persons, are found in the subject bibliography.

Some nutritional enteral or parenteral compositions containing amino acids, with complementary nutrients, are described in subject bibliography (for example, US 5504072, FR 2710244 and WO 93/13767), which differ from those of present invention in the amino acids formulae as well as in that no method is described on how to calculate the diets amino acids proportion, and in that the compositions revealed are not specific nutritions for elder people.

Furthermore, we have found in subject books some revision articles referring to methods being used to determine the amino acids substantially required by adult persons (Fuller M.F. and Garlok P.J. Annu. Rev. Nutr. 14, 217-241 (1994), Zello G.A. et al., J. Nutr. 125, 2907-2915 (1995). Traditionally, experiments based on a nitrogen balance were used. Recently, calculations of requirements have been conducted, based on other methods such as the plasmatic concentration of amino acids, the direct oxidzation of amino acids or the indicators of amino acids oxidization, however, present recommendations ar still based on the nitrogen balance in spite of its limitations.

Thus it is understood that providing of amino acids formulations for parenteral or enteral nutrition specifically developed for elder people still is therapeutic problem. The present invention provides a new procedure permitting the calculation of required concentration of each amino acid to be administered in an artificial diet for elder persons in acordance with the intracellular needs of each amino acid; it is another object of the present invention the own computation formulae for enteral and parenteral artificial diets for elder persons, an amino acids formulation being understood as the proportion of the differente amino acids in the diet.

The amount of each amino acid not contaminated by the effect of nutrition received during previous days, which is calculated from zero time extrapolation of amino acids concentration average values, obtained by not providing for several days, any food to old animals, will be designated from now on *USEFUL POOL* of each amino acid.

The amino acids formulations, object of the invention, are calculated based on the Conversion Factor and on the *USEFUL POOL* SIZE OF EACH AMINO ACID.

The Conversion Factor is provided by comparing the amount of amino acid administered during a given time to the arterial concentration of amino acids at the end of said period.

Description of the Invention

The present invention provides new amino acids formulations for elder persons characterized in that every 100 grams of total amino acids contain:

    11 to 17 gr. of glutamic acid;
    6 to 8 gr. of L-serine;
    4 to 10 gr. of glycine;
    2 to 3.50 gr. of L-hystidine;
    3.50 to 5 gr. of L-treonine;
    5 to 6.50 gr.of L-alanine;
    4.50 to 7 gr. of L-arginine;
    8.25 to 10 gr. of L-proline;
    1.50 to 4.50 gr. of L-tyrosine;
    5.50 to 7 gr. of L-valine;
    2 to 3.50 gr. of L-metionine;
    0.25 to 2.25 gr. of L-cisteine;
    5.75 to 7.25 gr. of L-isoleucine;
    9 to 10.75 gr. of L-leucine;
    4 to 6 gr. of L-fenilalanine;

1 to 2.50 gr. of L-triptophane;
7 to 8.50 gr. of L-lysine.

The present invention also provides a new procedure to calculate amino acids formulae, i.e., the required concentration of each amino acid to be administered in an artificial diet.

Also it is an object of the present invention the theraspeutic use of new amino acids formulations for the preparation of artificial parenteral or enteral diets for administration to elder persons.

Preferred amino acids formulations are those containing in every 100 grams of total amino acids the following;

11 to 12 gr. of L-glutamic acid
6.50 to 7.75 gr. of L-serine
8.75 to 9.75 gr. of glycine
2.50 to 3.50 gr. of L-hystidine
3.75 to 4.80 gr. of L-treonine
5.50 to 6.50 gr. of L-alanine
5.75 to 7 gr. of L-arginine
8.75 to 10 gr. of L-proline
1.50 to 2.75 gr. of L-tyrosine
5.80 to 7 gr. of L-valine
2.25 to 3.25 gr. of L-metionine
0.25 to 1.50 gr. of L-cisteine
6 to 7 gr. of L-isoleucine
9.50 to 10.60 gr. of L-leucine
4 to 5 gr. of L-fenilananine
1.25 to 2.30 gr. of L-triptophane
7.25 to 8.30 gr. of L-lysine;

which are used for the preparation of parenteral diets to be administered to elder persons, or those with every 100 grams containing:

16 to 17 gr. of L-glutamic acid
6.30 to 7.40 gr. of L-serine
4.30 to 5.30 gr. of glycine
2.30 to 3.30 gr. of L-hystidine
3.50 to 4.50 gr. of L-treonine
5.25 to 6.25 gr. of L-alanine
4.50 to 5.50 gr. of L-arginine
8.50 to 9.50 gr. of L-proline
3.25 to 4.30 gr. of L-tyrosine
5.50 to 6.60 gr. of L-valine
2 to 3 gr. of L-metionine
1.25 to 2.25 gr. of L-cisteine
5.80 to 6.80 gr. of L-isoleucine
9 to 10 gr. of L-leucine
5 to 6 gr. of L-fenilananine
1.25 to 2 gr. of L-triptophane
7 to 8 gr. of L-lysine;

which are used for the preparation of enteral diets to be administered to elder persons, that also can contain in every 100 grams of said latter formula 7 to 8 gr. of L-anserine and 0.50 to 1.50 gr. of L-aspartic acid.

It is particularly preferred those amino acids formulations in which every 100 grams of total amino acids contain the following:

11.2 to 11.6 gr. of L-glutamic acid
7 to 7.4 gr. of L-serine
9 to 9.4 gr. of glycine
2.8 to 3.2 gr. of L-hystidine
4.1 to 4.5 gr. of L-treonine

5.8 to 6.2 gr. of L-alanine

6.1 to 6.5 gr. of L-arginine

9.2 to 9.6 gr. of L-proline

1.9 to 2.3 gr. of L-tyrosine

6.2 to 6.6 gr. of L-valine

2.5 to 2.9 gr. of L-metionine

0.6 to 1 gr. of L-cisteine

6.4 to 6.8 gr. of L-isoleucine

9.9 to 10.3 gr. of L-leucine

4.3 to 4.7 gr. of L-fenilananine

1.6 to 2 gr. of L-triptophane

7.6 to 8 gr. of L-lysine;

which are used for the preparation of parenteral diets for elder persons.

It is also preferred those containing for every 100 grams of total amino acids the following:

16.4 to 16.8 gr. of L-glutamic acid

6.6 to 7 gr. of L-serine

4.6 to 5 gr. of glycine

2.6 to 3. gr. of L-hystidine

3.8 to 4.2 gr. of L-treonine

5.5 to 5.9 gr. of L-alanine

4.8 to 5.2 gr. of L-arginine

8.7 to 9.1 gr. of L-proline

3.6 to 4 gr. of L-tyrosine

5.9 to 6.3 gr. of L-valine

2.4 to 2.8 gr. of L-metionine

1.5 to 1.9 gr. of L-cisteine

6.1 to 6.5 gr. of L-isoleucine

9.4 to 9.8 gr. of L-leucine

5.3 to 5.7 gr. of L-fenilananine

1.5 to 1.9 gr. of L-triptophane

7.3 to 7.7 gr. of L-lysine;

used for the preparation of parenteral diets to be administered to elder persons, which can also contain for every 100 grams of the latter formula, 7.2 to 7.6 gr. of L-anserine and 0.7 to 1.1 gr. of L-aspartic acid.

As it has been described above, a second object of the present invention consists of providing a new procedure to calculate amino acids formulae. According to the present invention the amino acids formulae, i.e. the required concentration of each amino acid to be administered in an artificial diet for elder persons, are obtained through a procedure that uses elder animals, as described below.

The amount of each amino acid ($\mu$mol/day) to provide to the elder animal, for example a rat, is obtained from the Conversion Factor of each amino acid and the *USEFUL POOL* size.

$$[AA\ diet\ (\mu mol/day)] = Factor\ (1/day) \times [arterial\ or\ tissue\ AA\ (\mu mol/l)]$$

where *AA* means amino acid; *Factor* is the Conversion Factor; and [*arterial or tissue AA*] is the *USEFUL POOL* size for each amino acid in arterial blood or tissues.

If willing to give the percentages of amino acids concentrations, the amino acid fomulation in % can be provided (grams of free amino acids/100 grams of total amino acids) in the Parenteral or Enteral Basic Diet.

A final Amino Acids Formulation can be established from the Amino Acids Formulation calculated above, considering, for example, the stability studies of amino acid solutions, or the amino acids solubility in the case of Parenteral Nutrition, and the amino acids profile of proteins available in the case of Enteral Nutrition.

The *USEFUL POOL* of amino acids is determined from experiments carried out, for ethical reasons, on laboratory animals such as rats or similar. Since the requirements and metabolism of amino acids on mammals do not show substantial differences, we can extrapolate the results obtained from experiments carried out on animals onto human beings (see, for example, the article of H.N. Munro "Evolution of protein metabolism in mammals", published in Mammalian protein metabolism, vol. III, pag. 133-182, edited by H.N. Munro, Academic Press, New York, 1969).

The *USEFUL POOL* of amino acids based on identification of the amount of each amino acid (amino acids *POOL* size) not contaminated by the effect of nutrition received during previous days, can be calculated from the zero time extrapolation of average of amino acids concentracions values obtained by not providing, for a period between one day and the normal life of the animal used, any food to old animals, for example male Wistar rats 18 to 30 months old, and in the case of Wistar rats the maximum period will be 20 days.

Once the period of fast is over, blood samples can be obtained by means of puncturing, under the effects of anesthesia, the carotide artery, or tissue samples can be taken from different organs, such as liver, heart, kidneys or jejunum, of the sacrificed animal. Normally it is preferred to analyze the arterial blood as it is easily available and remains unaltered even with malnutrition (fast). The lack of nutrients alters the arterial flow of amino acids in a particular tissue, but keeps constant the arterial blood flow. After the malnutrition period, the flows of arterial blood amino acids are modified.

The arterial blood and the tissues are processed according to known methods and the analysis of amino acids is carried out according to usual cromatography methods. An analysis is performed with the values of amino acids concentrations obtained, for example of simple regression, by applying the minimum square method, thus obtaining the line:

$$Y = a + bX$$

wherein for each "X" value (days of fast) there is a series of "Y" values (amino acids concentrations). With this method, an average value of "Y" values (amino acids concentrations) can be estimated when "X" (days of fast) is zero (this value corresponds to the "a" estimated by square minimums or origin ordinate).

When applying the simple linear regression model to each amino acid three types of response can be found:

A) The value of amino acids decreases along the analysis period in a statistically significant pattern. In this case, the maximum expansion of the *USEFUL POOL* corresponds to day zero and to the origin ordinate and the minimum expansion of the *USEFUL POOL* can he calculated from the average of values obtained at the end of the analysis period, for example the 15th day.

B) The value of amino acids increases along the analysis period in a statistically significant pattern. In this case, the maxiumum expansion of the *USEFUL POOL* can he computed from the average of values obtained at the end of the analysis period, for example the 15th day, and the minimum expansion of the *USEFUL POOL* can be calculated on day zero, corresponding to the origin ordinate.

C) The amino acids are not altered by the effect of fast in a statistically significant pattern. In this case, the maximum and minimum expansion of the *USEFUL POOL* can be calculated as in cases A and B.

The *USEFUL POOL* is that to be reached at the end of the malnutrition period.

The *USEFUL POOL* for each amino acid is calculated from the average value of amino acids concentrations at the time zero plus two standrd deviations (2SD).

The Conversion Factor required to calculate the Amino Acids Formulation can be provided by comparing the amount of amino acid administered in a 1 to 5 day period, for example 24 hours in the case of a parenteral nutrition diet, or 4 days in the case of enteral nutrition diet, to the amino acids arterial concentration once that period hs elapsed:

$$Factor = (AA\ administered) / (arterial\ AA)$$

$$Factor = (\mu mol/day) / (\mu mol/l)$$

where *AA* means amino acid and *Factor* is the Conversion Factor.

Description of Examples of Embodiments

In order to better understand the object of the present invention, some examples of preferential practical embodiments are described referred to encloses figures, wherein:

Figure 1 represents a chart corresponding to the concentration of L-Triptophane in arterial blood showing how the concentration of said amino acid decreases in a statistically significant pattern (probability = 0.007).

Figure 2 shows a chart corresponding to the concentration of Glycine in arterial blood, showing how the concentration of said amino acid increases in a statistically significant pattern (probability = 0.00002).

Figure 3 shows a chart corresponding to the concentration of L-alanine in arterial blood, showing how the concentration of said amino acid decreases in a non ststisticaly significant pattern (probability = 0.4569).

The following examples ilustrate, without any limitations, the procedure used to calculate the required concentration of each amino acid to be administered in a artificial nutrition to elder persons, and how to provide in this manner the amino acids formulations for the preparation of either parenteral or enteral artificial nutritions.

**Example 1 : Formula for Amino Acids in Parenteral Nutrition Solutions. Use of Arterial Blood for its Determination.**

1A.- Calculation of amino acids *USEFUL POOL*

48 male 24 months old Wistar rats, weighing 501 to 923 grams, considered as old rats according to international standards, were distributed at random in 8 groups of 6 animals each. The rats were kept without any food for 1, 3, 5, 7. 11. 13 and 15 days respectively. The two year old rats stayed alive after 15 to 20 days. However, the maximum time of fast was established at 15 days so that most rats will stay alive till the day of sacrifice.

Once the time of fast established for each animal is over, blood samples are obtained, under the effects o anesthesia, by puncturing the carotide artery, and then the samples are centrifugated at 1.000° for 10 minutes and the floating matter is ultrafiltered for 30 minutes (filter grade 1.000). The ultra filtered samples are frozen at - 80°C until they are analysed.

The concentration of the different amino acids is analyzed by high resolution liquid cromatography (HPLC) (Pico-Tag Waters System), by means of pre column derivatization of samples with phenilsotiocionate (PITC) to form phenilthiocarbamilaminoacids (Bidligmeyer B.A et al, J. Chrom (Biomed Appl). 336, 93-104 (1984). The amount of sample inyected is 10 $\mu$l, using an automatic injector (Waters 700). The features of the column (PICO-TAG$^R$ Millipore, for phisiological amino acids) are 30 cm x 4.6 mm, $C_{18}$ (5 $\mu$m). The solving system used consists of a mixture of two diluents (A) an sodium acetate aqueous buffer pH 6.35, and (B) a mixture of acetonitrile, methanol and water. The internal standard used is metioinsulphone. The calibration curve includes following amino acids concentrations: 25, 50, 100, 200, 400 and 600 $\mu$mol/l. The analysis temperature is 46 °C. The time the cromatogram is 90 minutes. After that the system is balanced until next injection is made.

A simple regression analysis is made with the values obtained from amino acids concentrations. In order to obtain the best regression line the square minium method is applied, which provides the line:

$$Y = a + bX$$

For each "X" value (days of fast) there is a series of "Y" values (amino acids concentration). Thus, with this method it is estimated the average value of series of"Y" values (amino acids concentration) when "X" (days of fast) is zero (this value corresponds to "a" estimated by the square minimum or origin ordinate).

The amino acids concentration on different tissues can be related to the period of fast in a, or not, significant way. Although the lack of correlation may make it difficult to figure the theoretical amino acids concentration the day zero of fast as a value used as a reference point for the initial formulation of diet, still the computed "a" (origin ordinate) can be estimated (or even the average value of values obtained the day 15th of fast).

In practice, the pattern found varies not only with respect to the analysed amino acid but also with respect to the analyzed tissue. After application of the simple linear regression model three types of response are found:

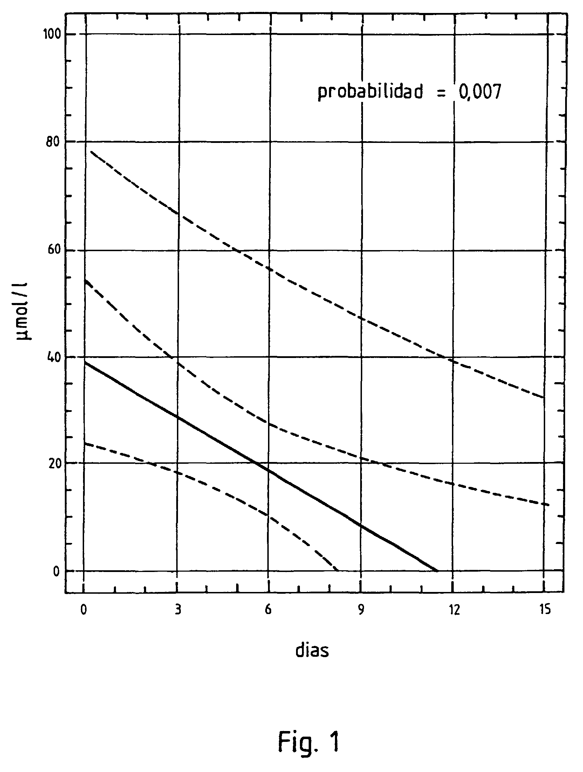

A) A group of amino acids decreases its value along the study period in a statistically significant way (for example, the amino acid L-triptophane in arterial blood, see figure 1).

In this case, coefficient "a", ($\mu$M amino acid the day zero) is used to define the maximum expansion of *USEFUL POOL* size for each amino acid in the particular organ analyzed, in this case the arterial blood and values provided on day 15th of fast the minimum expansion of *USEFUL POOL* .

In that case, the maximum expansion of *USEFUL POOL* happens on day zero corresponding to the estimated origin ordinate with a value of 38.94 ± 7.18 $\mu$mol/l.

The minimum expansion of *USEFUL POOL* is computed from average of values obtained the day 15th of fast, with a value of 0 $\mu$mol/l.

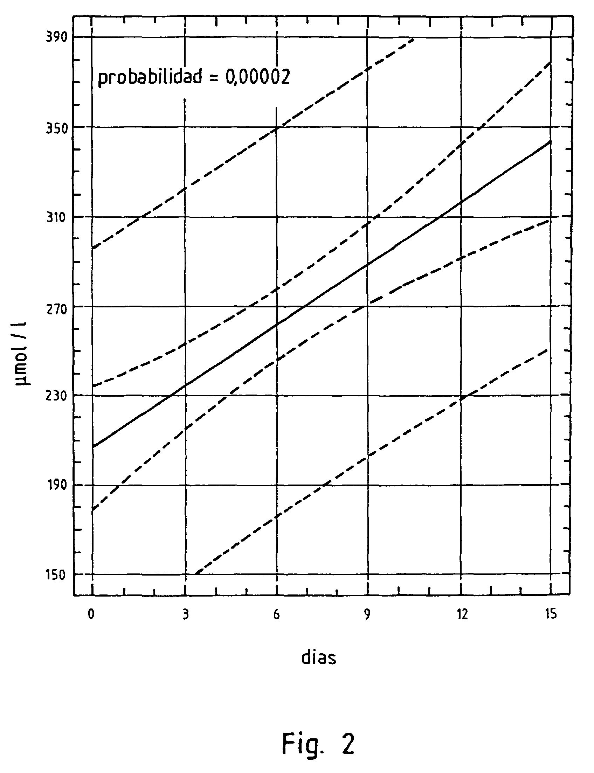

B) Another group of amino acids shows a trend to increase with fast in a significant way (for example the amino acid glycine in arterial blood, see figure 2).

In this case, the average of values provided on day 15th of fast is identified as the maximum expansion for the *USEFUL POOL* size for each amino acid in the organ analyzed, in this case arterial blood, and the coefficient "a"

(μM amino acid the day zero) is defined as the minimum expansion of *USEFUL POOL*.

In that case, the maximum expansion of *USEFUL POOL* is computed from the average of values obtained on the day 15th of fast, having a value of 344.24 ± 13.45 μmol/l.

The minimum expansion of *USEFUL POOL* is provided on the day zero and corresponds to origin ordinate, having a value of 206.68 ± 13.45 μmol/l.

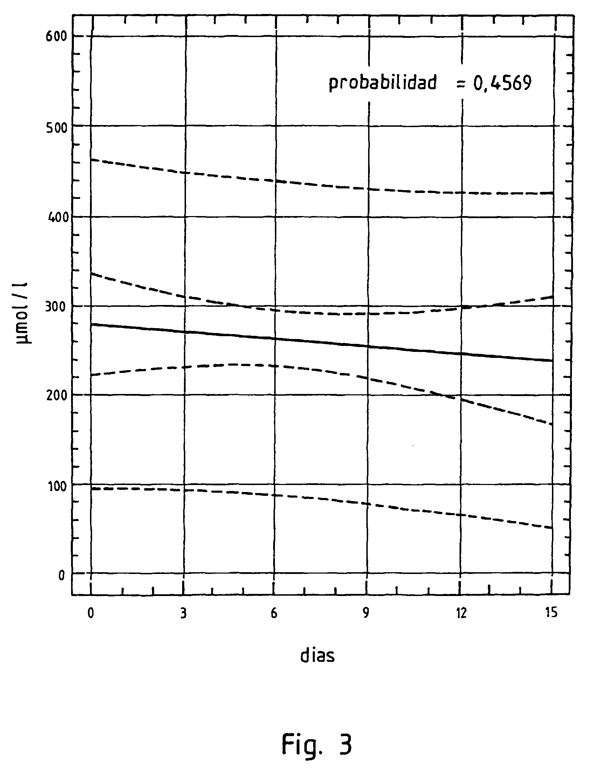

C) Finally, the remaining amono acids do not seem to be altered by the effect of fast in a significant way (for example, amino acid L-alanine in arterial blood, see figure 3).

In these cases the regression line runs in parallel with X axis. However, as it was said before the maximum and minimum expansion *POOL* can be computed as previously described (see A and B). In case of statistical significance, there appear differences in the maximum expansion of *POOL* size, provided on day zero if the slope decreases or in the day 15th when the slope rises. At the same time te minimum expansion is reached the day zero if the slope rises and on day 15th when the slope decreases.

In this case, the maximum expansion of *USEFUL POOL* is provided on day zero corresponding to the estimated origin ordinate having a value of 278.79 ± 27.42 μmol/l.

The minimum expansion of *USEFUL POOL* is computed from the average of values obtained on the day 15th of fast, having a value of 238.09 ± 53.00 μmol/l.

In a similar way the charts corresponding to the remaining amino acids: glutamic acid, serine, hystidine, treonine, arginine, proline, tyrosine, valine, metionine, cisteine, isoleucine, leucine, phenilalanine and lysine are provided.

From data provided by the simple linear regression analysis the *USEFUL POOL* is considered as the average of concentration values obtained at zero time plus two standard deviations (table 1).

The *USEFUL POOL* has to be reached after administering the nutrition. With the nutrition, the amino acids increasing with fast return to their usual values and vice verasa, those decreasing reah usual values after provision of food. Finally, those amino acids not altered stay without significant changes after nutrition.

Table 1 shows the computing of *USEFUL POOL*: the average of amino acids concentrations extrapolated to zero time plus two standard deviations (2SD).

Table 1

| Computing the *USEFUL POOL* | | | |
| --- | --- | --- | --- |
| Amino acid | Average (μmol/l) | 2 SD | Useful pool (μmol/l) |
| L-Glutamic Acid | 153.3 | 40 | 193.3 |
| L-Serine | 208.6 | 40 | 248.6 |
| Glycine | 206.7 | 27 | 233.7 |
| L-Hystidine | 47.2 | 8 | 55.2 |
| L-Treonine | 281.7 | 45 | 326.7 |
| L-Alanine | 278.8 | 60 | 338.8 |
| L-Arginine | 93.5 | 27 | 120.5 |
| L-Proline | 122.0 | 17 | 139.0 |
| L-Tyrosine | 64.0 | 10 | 74.0 |
| L-Valine | 146.0 | 22 | 168.0 |
| L-Metionine | 42.8 | 7 | 49.8 |
| L-Cisteine | 140.3 | 16 | 156.3 |
| L-Isoleucine | 66.09 | 10 | 76.09 |
| L-Leucine | 134.8 | 20 | 154.8 |
| L-Phenilananine | 63.3 | 12 | 75.3 |
| L-Triptophane | 38.9 | 13 | 51.9 |
| L-Lysine | 239.8 | 30 | 269.8 |

1B. Computing the Conversion Factor

The conversion factor is obtained by comparing the amount of amino acid (AA) administered (μmol/day) to the arterial concentration provided by amino acids (μmol/l) in 24 months old male Wistar rats.

To compute the conversion factor of a parenteral nutrition diet the parenteral nutrition is supplied for 24 hours and the arterial concentration determined after said period.

$$Factor = (AA\ administered)/(arterial\ AA)$$

$$Factor = (\mu mol/day\ adm.)/(\mu mol$$

Table 2 provides the Final Conversion Factor for each amino acid in a parenteral nutrition.

Table 2

| Final Conversion Factor in a Parenteral Diet | |
|---|---|
| Amino acid | Factor (l/day) |
| L-Glutamic Acid | 6.54 |
| L-Serine | 2.47 |
| Glycine | 4.64 |
| L-Hystidine | 6.11 |
| L-Treonine | 2.40 |
| L-Alanine | 4.62 |
| L-Arginine | 2.19 |
| L-Proline | 4.68 |
| L-Tyrosine | 0.21 |
| L-Valine | 3.94 |
| L-Metionine | 5.98 |
| L-Cisteine | 0.93 |
| L-Isoleucine | 11.91 |
| L-Leucine | 7.50 |
| L-Phenilalanine | 6.82 |
| L-Triptophane | 3.07 |
| L-Lysine | 1.83 |

1C. Computing the Formula for Amino Acids

The amount of each amino acid (μmol/day) requirted by the rat is figured using the formula of 1B (see Table 3), i.e., from the Conversion Factor and the *USEFUL POOL* size of each amino acid.

$$[AA\ diet\ (\mu mol/day)] = Factor\ (l/day)\ x\ [Arterial\ AA\ (\mu mol/l)]$$

Table 3

| Amino acid | Factor (l/day) | Useful pool AA (μmol/l) | Diet AA (μmol/day) |
|---|---|---|---|
| L-Glutamic Acid | 6.54 | 193.3 | 1,264.18 |

Table 3 (continued)

| Amino acid | Factor (l/day) | Useful pool AA (µmol/l) | Diet AA (µmol/day) |
|---|---|---|---|
| L-Serine | 2.47 | 248.66 | 614.04 |
| Glycine | 4.64 | 233.7 | 1,084.44 |
| L-Hystidine | 6.11 | 55.2 | 337.27 |
| L-Treonine | 2.40 | 326.7 | 784.05 |
| L-Alanine | 4.62 | 338.8 | 1,565.60 |
| L-Arginine | 2.19 | 120.5 | 263.93 |
| L-Proline | 4.68 | 139.0 | 650.52 |
| L-Tyrosine | 0.21 | 74.0 | 15.54 |
| L-Valine | 3.94 | 168.0 | 661.92 |
| L-Metionine | 5.98 | 49.8 | 293.02 |
| L-Cisteine | 0.93 | 156.3 | 145.08 |
| L-Isoleucine | 11.91 | 76.1 | 905.16 |
| L-Leucine | 7.50 | 154.8 | 1,155.61 |
| L-Phenilananine | 6.82 | 75.3 | 511.50 |
| L-Triptophane | 3.07 | 51.9 | 159.64 |
| L-Lysine | 1.83 | 269.8 | 494.10 |

Table 4 provides the amino acids concentrations as percentages so that the % Amino acid formulation will be obtained (grams of free amino acids/100 grams of total amino acids) in the Parenteral Basic Diet.

Table 4

| Amino acid formulation % in Parenteral basic Diet | | | |
|---|---|---|---|
| Amino acid | Diet AA (µmol/day) | % mol/100 molAA | Basic Diet % g of AA |
| L-Glutamic Acid | 1,264.18 | 11.59 | 13.89 |
| L-Serine | 614.04 | 5.63 | 4.82 |
| Glycine | 1,084.44 | 9.94 | 6.08 |
| L-Hystidine | 337.27 | 3.09 | 3.91 |
| L-Treonine | 784.05 | 7.19 | 6.97 |
| L-Alanine | 1,565.60 | 14.35 | 10.41 |
| L-Arginine | 263.93 | 2.42 | 3.43 |
| L-Proline | 650.52 | 5.97 | 5.59 |
| L-Tyrosine | 15.54 | 0.14 | 0.21 |
| L-Valine | 661.92 | 6.07 | 5.79 |
| L-Metionine | 293.02 | 2.69 | 3.26 |
| L-Cisteine | 145.08 | 1.33 | 1.31 |
| L-Isoleucine | 905.16 | 8.30 | 8.87 |
| L-Leucine | 1,155.61 | 10.59 | 11.31 |
| L-Phenilananine | 511.50 | 4.69 | 6.31 |
| L-Triptophane | 159.64 | 1.46 | 2.43 |

Table 4 (continued)

| Amino acid formulation % in Parenteral basic Diet | | | |
|---|---|---|---|
| Amino acid | Diet AA ($\mu$mol/day) | % mol/100 molAA | Basic Diet % g of AA |
| L-Lysine | 494.10 | 4.53 | 5.39 |

1D. Formula for Amino Acids in Parenteral Nutrition Solutions

After having conducted stability studies on solutions and having in consideration that the solubility of the various amino acids and the production process, it is established that the Parenteral Nutrition Diet contains the Formula for Amino Acids shown in Table 5 [NPT Diet (%)].

Table 5

| Formula for Amino Acids in Parenteral Diet | | |
|---|---|---|
| Amino acid | Basic Diet NPT | Diet NPT(%)* |
| L-Glutamic Acid | 13.89 | 11.43 |
| L-Serine | 4.82 | 7.21 |
| Glycine | 6,08 | 9.26 |
| L-Hystidine | 3.91 | 3.02 |
| L-Treonine | 6.97 | 4.29 |
| L-Alanine | 10.41 | 5.99 |
| L-Arginine | 3.43 | 6.34 |
| L-Proline | 5.59 | 9.40 |
| L-Tyrosine | 0.21 | 2.18 |
| L-Valine | 5.79 | 6.43 |
| L-Metionine | 3.26 | 2.76 |
| L-Cisteine | 1.31 | 0.80 |
| L-Isoleucine | 8.87 | 6.61 |
| L-Leucine | 11,31 | 10.15 |
| L-Phenilananine | 6.31 | 4.50 |
| L-Triptophane | 2.43 | 1.80 |
| L-Lysine | 5.39 | 7.86 |

\* Grams of free amino acids / 100 g. of total amino acids

Ejemplo 2: Formula for Amino Acids in Enteral Nutrition solutions. Use of arterial blood for determination of said ormula.

2A. Computing the Conversion factor

Following the procedure described in paragraph 1B of example 1, but in this case providing a defined enteral nutrition for 4 days and determining the arterial concentration of amino acids after that period.

$$\text{Factor} = [\text{AA administered}] / [\text{arterial AA}]$$

$$\text{Factor} = [\mu\text{mol/day}] / [\mu\text{mol/l}]$$

Table 6 shows the Final Conversion Factor for eacho amino acid in an enteral nutrition.

Table 6

| Final Conversion Factor in an enteral Nutrition | |
|---|---|
| Amino acid | Factor (l/day) |
| L-GlutamicAcid | 43.19 |
| L-Serine | 10.70 |
| Glycine | 8.83 |
| L-Hystidine | 16.86 |
| L-Treonine | 4.20 |
| L-Alanine | 4.22 |
| L-Arginine | 8.06 |
| L-Proline | 13.73 |
| L-Tyrosine | 18.46 |
| L-Valine | 14.44 |
| L-Metionine | 11.26 |
| L-Cisteine | 1.63 |
| L-Isoleucine | 12.95 |
| L-Leucine | 24.19 |
| L-Phenilananine | 19.86 |
| L-Triptophane | 10.80 |
| L-Lysine | 9.075 |

2B. Computing a Formula for Amino Acids

Following the procedure described in paragraph 1C of example 1, we obtain the amount of amino acids ($\mu$mol/day) to be supplied to the rat (see Table 7).

Table 7

| Amino acid | Factor | Useful pool of AA ($\mu$mol/l) | AAc Diet ($\mu$mol/day) |
|---|---|---|---|
| L-GlutamicAcid | 43.19 | 193.3 | 8,348.6 |
| L-Serine | 10.70 | 248.6 | 2,660.0 |
| Glycine | 8.83 | 233.7 | 2,063.6 |
| L-Hystidine | 16.86 | 55.2 | 930.6 |
| L-Treonine | 4.20 | 326.7 | 1,372.1 |
| L-Alanine | 4.22 | 338.8 | 1,429.7 |
| L-Arginine | 8.06 | 120.5 | 971.2 |
| L-Proline | 13.73 | 139.0 | 1,908.4 |
| L-Tyrosine | 18.46 | 74.0 | 1,366.0 |
| L-Valine | 14.44 | 168.0 | 2,425.9 |

Table 7 (continued)

| Amino acid | Factor | Useful pool of AA (μmol/l) | AAc Diet (μmol/day) |
|---|---|---|---|
| L-Metionine | 11.26 | 49.8 | 551.7 |
| L-Cisteine | 1.63 | 156.3 | 254.3 |
| L-Isoleucine | 12.95 | 76.09 | 984.2 |
| L-Leucine | 24.19 | 154.8 | 3,725.3 |
| L-Phenilananine | 19.86 | 75.3 | 1,489.5 |
| L-Triptophane | 10,80 | 51.9 | 561.6 |
| L-Lysine | 9,075 | 269.8 | 2,450.2 |
| TOTAL | | | 33,493.3 |

Table 8 shows the amino acids concentrations in percentages, providing a Amino acid formulation in % (grams of free amino acids / 100 g. of total amino acids) in the Enteral Basic Diet.

Table 8

| Amino acid formulation in % in Enteral Basic Diet | | | |
|---|---|---|---|
| Amino acid | Diet of AA (μmol/day) | % mol/100 mol AA | Basic Diet % gr. of AA |
| L-GlutamicAcid | 8,348.6 | 24.93 | 27.49 |
| L-Serine | 2,660.0 | 7.94 | 6.26 |
| Glycine | 2,063.6 | 6.16 | 3.47 |
| L-Hystidine | 930.6 | 2.78 | 3.23 |
| L-Treonine | 1,372.1 | 4.10 | 3.66 |
| L-Alanine | 1,429.7 | 4.27 | 2.85 |
| L-Arginine | 971.28 | 2.90 | 3.79 |
| L-Proline | 1,908.4 | 5.70 | 4.92 |
| L-Tyrosine | 1,366.0 | 4.08 | 5.54 |
| L-Valine | 2,425.9 | 7.24 | 6.36 |
| L-Metionine | 551.7 | 1.65 | 1.84 |
| L-Cisteine | 254.3 | 0.76 | 0.69 |
| L-Isoleucine | 984.2 | 2.94 | 2.89 |
| L-Leucine | 3,725.3 | 11.12 | 10.94 |
| L-Phenilananine | 1,489.5 | 4.45 | 5.51 |
| L-Triptophane | 561.6 | 1.68 | 2.57 |
| L-Lysine | 2,450.2 | 7.32 | 8.02 |
| TOTAL | 33,493.3 | 100 | 100 |

2C. Amino acid formulation in Enteral Nutrition solutions.

The Enteral Nutrition Diet should contain the Amino acid formulation of Table 9 [proposed N. Enteral diet (%)] based on the Amino acid formulation provided for an Enteral Basic Diet and considering the amino acid profile of proteins available in the market and having into account that in this case the amino acids will be supplied as proteins, possibly being totally hydrolized. Also, the enteral diet might contain othr amino acids (for example L-anserine and L-aspartic acid) depending on the proteins supplied.

Table 9

| Amino acid formulation in Enteral Diet | | |
|---|---|---|
| Amino acid | Basic Diet N. Enteral (%)* | Proposed Diet N.enteral (%)* |
| L-GlutamicAcid | 27.49 | 16.59 |
| L-Serine | 6.26 | 6.86 |
| Glycine | 3.47 | 4.82 |
| L-Hystidine | 3.23 | 2.87 |
| L-Treonine | 3.66 | 4.06 |
| L-Alanine | 2.85 | 5.72 |
| L-Arginine | 3.79 | 5.08 |
| L-Proline | 4.92 | 8.96 |
| L-Tyrosine | 5.54 | 3.80 |
| L-Valine | 6.36 | 6.14 |
| L-Metionine | 1.84 | 2.62 |
| L-Cisteine | 0.69 | 1.75 |
| L-Isoleucine | 2.89 | 6.31 |
| L-Leucine | 10.94 | 9.66 |
| L-Phenilananine | 5.51 | 5.51 |
| L-Triptophane | 2.57 | 1.71 |
| L-Lysine | 8.02 | 7.54 |

* Grams of free amino acids / 100 g. of total amino acids Proposed diet: 7.45 gr. of L-anserine and 0.91 gr. of acid L-aspartic per 100 gr. of amino acids.

Example 3. Amino acid formulation in Parenteral Nutrition solutions. Use of jejunem for determination of said formula.

The procedure described in the example 1 will be followed but the jejunem will be used instead of arterial blood.

The jejunem sample is provided by removing the first 30 cms. from the pylorus and cutting out a 20 cms. fragment under a 3.5 grs. stress. The serose is separated by longitudinal sectioning of intestine and by grazing the lumen surface with a slide. The material provided by grazing (muscles and mucose) is deposited in a essay glass and weighed. The tissue sample is weighed, then frozen in liquid nitrogen ad stored at - 80 °C till be processed.

The tissue treatment is made in accordance with the method Adibi S.A. Am. J. Physiol, 221, 3, 829-838 (1971). An aliquote taken from the tissue analyzed is weighed and homogenized with sulphosalicilic acid at 6% (1 ml/100 mg of tissue) at 4°C. The floating material provided is ultra filtered for 30 minutes (cut 10000) and frozen till it is analyzed.

The measurement of tissue proteins is carried out by the method Lowry O.H. J. Biol. Chem, 193, 265-275 (1951).

Example 4: Amino acid formulation in Enteral Nutrition solutions. Use od jejunem for determination of said formula.

The procedure described in the example 2 will be followed except that jejunem will be used instead of arterial blood. The tissue treatment is carried out as described in example 3.

**Claims**

1. Amino acid formulation for elder persons characterized in that: it contains for every 100 grams of amino acids

11 to 17 grams of L-glutamic acid
6 to 8 grams of L-serine
4 to 10 grams of glycine
2 to 3.50 grams of L-hystidine
3.50 to 5 grams of L-tronine
5 to 6.50 grams of L-alanine
4.50 to 7 grams of L-arginine
8.25 to 10 grams of L-proline
1.50 to 4.50 grams of L-tyrosine
5.50 to 7 grams of L-valine
2 to 3.50 grams of L-metionine
0.25 to 2.25 grams of L-cisteine
5.75 to 7.25 grams of L-isoleucine
9 to 10.75 grams of L-leucine
4 to 6 grams of L-phenilananine
1 to 2.50 grams of L-triptophane
7 to 8.50 grams of L-lysine.

2. Amino acid formulation according to claim 1 wherein every 100 grams of amino acids contain:

11 to 12 grams of L-glutamic acid
6.50 to 7.75 grams of L-serine
8.75 to 9.75 grams of glycine
2.50 to 3.50 grams of L-hystidine
3.75 to 4.80 grams of L-treonine
5.50 to 6.50 grams of L-alanine
5.75 to 7 grams of L-arginine
8.75 to 10 grams of L-proline
1.50 to 2.75 grams of L-tyrosine
5.80 to 7 grams of L-valine
2.25 to 3.25 grams of L-metionine
0.25 to 1.50 grams of L-cisteine
6 to 7 grams of L-isoleucine
9.50 to 10.60 grams of L-leucine
4 to 5 grams of L-phenilananine
1.25 to 2.30 grams of L-triptophane
7.25 to 8.30 grams of L-lysine.

3. Amino acid formulation accoding to claim 1 wherein every 100 grams of amino acids contain:

16 to 17 grams of L-glutamic acid
6.30 to 7.40 grams of L-serine
4.30 to 5.30 grams of glycine
2.30 to 3.30 grams of L-hystidine
3.50 to 4.50 grams of L-treonine
5.25 to 6.25 grams of L-alanine
4.50 to 5.50 grams of L-arginine
8.50 to 9.50 grams of L-proline
3.25 to 4.30 grams of L-tyrosine
5.50 to 6.60 grams of L-valine
2 to 3 grams of L-metionine
1.25 to 2.25 grams of L-cisteine
5.80 to 6.80 grams of L-isoleucine
9 to 10 grams of L-leucine
5 to 6 grams of L-phenilananine
1.25 to 2 grams of L-triptophane
7 to 8 grams of L-lysine.

4. Amino acid formulation according to claim 3 also containing, for every 100 grams of amino acids, 7 to 8 grams of L-anserine and 0.50 to 1.50 grams of L-aspartic acid.

5. Amino acid formulation according to claim 2 wherein every 100 grams of total amino acids contain:

11.2 to 11.6 grams of L-glutamic acid
7 to 7.4 grams of L-serine
9 to 9.4 grams of glycine
2.8 to 3.2 grams of L-hystidine
4.1 to 4.5 grams of L-treonine
5.8 to 6.2 grams of L-alanine
6.1 to 6.5 grams of L-arginine
9.2 to 9.6 grams of L-proline
1.9 to 2.3 grams of L-tyrosine
6.2 to 6.6 grams of L-valine
2.5 to 2.9 grams of L-metionine
0.6 to 1 grams of L-cisteine
6.4 to 6.8 grams of L-isoleucine
9.9 to 10.3 grams of L-leucine
4.3 to 4.7 grams of L-phenilananine
1.6 to 2 grams of L-triptophane
7.6 to 8 grams of L-lysine.

6. Amino acid formulation according to claim 3 wherein every 100 grams of total amino acids contain:

16.4 to 16.8 grams of L-glutamic acid
6.6 to 7 grams of L-serine
4.6 to 5 grams of glycine
2.6 to 3 grams of L-hystidine
3.8 to 4.2 grams of L-treonine
5.5 to 5.9 grams of L-alanine
4.8 to 5.2 grams of L-arginine
8.7 to 9.1 grams of L-proline
3.6 to 4 grams of L-tyrosine
5.9 to 6.3 grams of L-valine
2.4 to 2.8 grams of L-metionine
1.5 to 1.9 grams of L-cisteine
6.1 to 6.5 grams of L-isoleucine
9.4 to 9.8 grams of L-leucine
5.3 to 5.7 grams of L-phenilananine
1.5 to 1.9 grams of L-triptophane
7.3 to 7.7 grams of L-lysine.

7. Amino acid formulation according to claim 4 further containing 7.2 to 7.6 grams of L-anserine and 0.7 to 1.1 grams of L-aspartic acid.

8. A procedure to calculate the amino acid formulation to be administered in a parenteral or enteral diet to elder persons characterized in that the amount of each amino acid ($\mu$mol/day) to receive is obtained from the Conversion Factor for each amino acid, understood as the amount of each amino acid not contaminated by the effect of nutrition provided during previous days, according to the formula:

$$[\text{Diet AA } (\mu\text{mol/day})] = \text{Factor (l/day)} \times [\text{arterial or tissue AA } (\mu\text{mol/l})]$$

wherein *AA* means amino acid; *Factor* is the Conversion Factor; and *[Arterial or tissue AA]* is the *USEFUL POOL* size of each amino acid in arterial blood or tisue.; Wherein the *USEFUL POOL* size is computed by extrapolating on zero time the average of concentration values of each amino acid obtained after having some old animals not fed for a period between one day and the survival average time specific to the type of animal use, and analyzing the amino acids content from blood samples or different tissues, through a simple regression analysis based on concentration values of amino acids provided, according to the line:

$$Y = a + bX$$

wherein for each "X" value (days of fast) there is a series of "Y" values (amino acids concentration); and wherein the Conversion Factor is provided by comparing the amount ofamino acids administered for a time period, preferably from one to 5 days, preferably 24 hours in the case of a parenteral nutrition diet or preferably 4 days in the case of an enteral nutrition diet, to arterial concentration of amino acids after said period, in accordance with the formulations:

$$Factor = [administered\ AA] / [arterial\ AA]$$

$$Factor = [\mu mol/day] / [\mu mol/l]$$

9. A procedure for computing the amino acid formulation to be administered in a parenteral or enteral diet to elder persons, according to claim 8, characterized in that the Final AminoAacid Formulation is established based on the Amino Acid Formulation computed having in consideration the stability studies carried out on amino acids solutions and amino acids solubility in case o a Parenteral Nutrition, and the amino acids profile of proteins available in the market in the ease of an enteral Nutrition.

10. Use of the amino acid formulation according to claim 1, with free or saline amino acids, of proteins or partially hydrolyzed proteins, for the preparation of an artificial diet to be administered to elder persons.

11. Use of the amino acid formulation according to claims 2 or 5, with free or aline amino aids, for the preparation of an artificial diet for parenteral administration to elder persons.

12. Use of the amino acid formulation according to claims 3, 4, 6 or 7, with proteins or partially hydrolized proteins, for the preparation of an artificial diet for enteral administration to elder persons.

Fig. 1

probabilidad = 0,00002

μmol / l

dias

Fig. 2

probabilidad = 0,4569

Fig. 3

# EP 0 917 826 A1

<table>
<tr><td colspan="2" align="center">**INTERNATIONAL SEARCH REPORT**</td><td>International application No.<br>PCT/ ES 98/00149</td></tr>
</table>

**A. CLASSIFICATION OF SUBJECT MATTER**

IPC 6    A23L 1/305

According to International Patent Classification (IPC) or to both national classification and IPC

**B. FIELDS SEARCHED**

Minimum documentation searched (classification system followed by classification symbols)

IPC 6    A23L 1/305, 1/30, 1/305B

Documentation searched other than minimum documentation to the extent that such documents are included in the fields searched

Electronic data base consulted during the international search (name of data base and, where practicable, search terms used)

EPODOC, WPI, PAJ. STN: CA EMBASE BIOSIS MEDLINE

**C. DOCUMENTS CONSIDERED TO BE RELEVANT**

| Category* | Citation of document, with indication, where appropriate, of the relevant passages | Relevant to claim No. |
|---|---|---|
| X | EP 0399656 A (KYOWA HAKKO KOGYO KABUSHIKI KAISHA) 28 November 1990 (28.11.90) Description; claim 6 | 1-12 |
| X | US 5221668 A (MARY F. HENNINGFIELD, et al) 22 June 1993 (22.06.93) Description; claim 6 | 1-12 |
| X | EP 0614616 A (CLINTEC NUTRITION COMPANY, AN ILLINOIS PARTENERSHIP) 14 September 1994 (14.09.94) page 9, lines 35-38; claim 19 | 1-12 |
| D,X | US 5504072 A (MARY K. SCHMIDL et al) 02 April 1996 (02.04.96) Description, claim 12 | 1-12 |
| A | EP 0721742 A (CLINTEC NUTRITION COMPANY, AN ILLINOIS PARTENERSHIP) 17 July 1996 (17.07.96) the whole document | 10-12 |

[X] Further documents are listed in the continuation of Box C.    [X] See patent family annex.

*   Special categories of cited documents:

"A"   document defining the general state of the art which is not considered to be of particular relevance

"E"   earlier document but published on or after the international filing date

"L"   document which may throw doubts on priority claim(s) or which is cited to establish the publication date of another citation or other special reason (as specified)

"O"   document referring to an oral disclosure, use, exhibition or other means

"P"   document published prior to the international filing date but later than the priority date claimed

"T"   later document published after the international filing date or priority date and not in conflict with the application but cited to understand the principle or theory underlying the invention

"X"   document of particular relevance; the claimed invention cannot be considered novel or cannot be considered to involve an inventive step when the document is taken alone

"Y"   document of particular relevance; the claimed invention cannot be considered to involve an inventive step when the document is combined with one or more other such documents, such combination being obvious to a person skilled in the art

"&"   document member of the same patent family

| Date of the actual completion of the international search | Date of mailing of the international search report |
|---|---|
| 31 August 1998 (31.08.98) | 28 September 1998 (28.09.98) |

| Name and mailing address of the ISA/<br>     S.P.T.O | Authorized officer |
|---|---|
| Facsimile No. | Telephone No. |

Form PCT/ISA/210 (second sheet) (July 1992)

## INTERNATIONAL SEARCH REPORT

| International application No. |
| --- |
| PCT / ES 98/00149 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | | |
| D,A | WO 9313767 A (NEW ENGLAND DEACONESS HOSPITAL CORPORATION) 22 July 1993 (22.07.93) | |
| D,A | FR 2710244 A (URSO MICHEL) 31 March 1995 (31.03.95) | |
| A | ES 2086460 A (MILUPA GmbH & CO, KG) 01 July 1996 (01.07.96) | |

------------------------

Form PCT/ISA/210 (continuation of second sheet) (July 1992)

EP 0 917 826 A1

<table>
<tr><td><b>INTERNATIONAL SEARCH REPORT</b></td><td>International application No.<br>PCT/ES 98 / 00149</td></tr>
</table>

**Box I    Observations where certain claims were found unsearchable (Continuation of item 1 of first sheet)**

This international search report has not been established in respect of certain claims under Article 17(2)(a) for the following reasons:

1. [X] Claims Nos.: **8 and 9**
because they relate to subject matter not required to be searched by this Authority, namely:

       **Concerning a mathematical method**

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II    Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**    [ ] The additional search fees were accompanied by the applicant's protest.
                     [ ] No protest accompanied the payment of additional search fees.

Form PCT/ISA/210 (continuation of first sheet (1)) (July 1992)

## INTERNATIONAL SEARCH REPORT
Information on patent family members

| International Application No |
|---|
| PCT / ES 98/ 00149 |

| Patent document cited in search report | Publication date | Patent familiy member(s) | Publication date |
|---|---|---|---|
| EP 0399656 | 28.11.1990 | US 5102871 | 07.04.1992 |
| | | DE 69006395 | 01.06.1994 |
| | | CA 2015186 | 24.10.1990 |
| | | AU 5382190 | 25.10.1990 |
| | | AU 624942 | 25.06.1992 |
| | | CN 1049598 | 06.03.1991 |
| | | JP 3139264 | 13.06.1991 |
| US 5221668 | 22.06.1993 | JP 2644086 | 25.08.1997 |
| | | MX 9300999 | 11.09.1993 |
| | | WO 9316595 | 22.06.1993 |
| | | NZ 249392 | 26.01.1996 |
| | | EP 0630181 | 28.12.1994 |
| | | CA 2128078 | 21.09.1993 |
| | | AU 3614393 | 13.09.1993 |
| | | JP 7500348 | 12.01.1995 |
| EP 0614616 | 14.09.1994 | US 5571783 | 05.11.1996 |
| | | CA 2116947 | 10.09.1994 |
| | | AU 5763094 | 15.09.1994 |
| | | JP 7048270 | 21.02.1995 |
| US 5504072 | 22.09.1996 | US 5438042 | 11.08.1995 |
| | | CA 2133783 | 09.04.1995 |
| EP 0721742 | 17.07.1996 | US 5686429 | 11.11.1997 |
| | | US 5589468 | 31.12.1996 |
| | | CA 2166003 | 14.07.1996 |
| | | AU 4076595 | 25.07.1996 |
| | | JP 8231411 | 10.09.1996 |
| WO 9313767 | 22.07.1993 | US 5278149 | 11.01.1994 |
| | | AU 3475193 | 03.08.1993 |
| FR 2710244 | 31.03.1995 | LU 88371 | 01.04.1994 |
| ES 2086460 | 01.07.1996 | EP 0488078 | 03.06.1992 |
| | | DE 4037447 | 27.05.1992 |
| | | AT 33836 | 15.02.1996 |

Form PCT/ISA/210 (patent family annex) (July 1992)